# EUROPEAN PATENT SPECIFICATION

(11) **EP 4 448 172 B1**
(45) Date of publication and mention of the grant of the patent: **29.07.2026**
(21) Application number: 22908477.7
(22) Date of filing: 16.12.2022
(51) Int. Cl.: B01J 31/24, C07B 35/02, C07B 53/00, C07F 15/06, B01J 31/22

(54) **METHODS OF MAKING CATIONIC COBALT COMPLEXES FOR ASYMMETRIC HYDROGENATION**
VERFAHREN ZUR HERSTELLUNG VON KATIONISCHEN KOBALTKOMPLEXEN ZUR ASYMMETRISCHEN HYDRIERUNG
PROCÉDÉS DE PRODUCTION DE COMPLEXES DE COBALT CATIONIQUES POUR HYDROGÉNATION ASYMÉTRIQUE

(30) Priority: 16.12.2021 US 202163290157 P
(43) Date of publication of application: 23.10.2024
(73) Proprietor: The Trustees of Princeton University, Princeton, NJ 08540 (US)
(72) Inventor: CHIRIK, Paul, J., Princeton, NJ 08540 (US); MACNEIL, Connor, S., Princeton, NJ 08540 (US)
(74) Representative: Greenwoods
(86) International application number: PCT/US2022/053117
(87) International publication number: WO 2023/114458

(56) References cited:
- US-A1- 2013 281 747
- US-A1- 2013 281 747
- US-B2- 9 463 451
- HONGYU ZHONG ET AL: "Syntheses and Catalytic Hydrogenation Performance of Cationic Bis(phosphine) Cobalt(I) Diene and Arene Compounds", ANGEWANDTE CHEMIE, VERLAG CHEMIE, HOBOKEN, USA, vol. 58, no. 27, 27 May 2019 (2019-05-27), pages 9194 - 9198, XP072103204, ISSN: 1433-7851, DOI: 10.1002/ANIE.201903766
- STEFAN C MEIER ET AL: "Access to the Bis-benzene Cobalt(I) Sandwich Cation and its Derivatives: Synthons for a "Naked" Cobalt(I) Source?", ANGEWANDTE CHEMIE, VERLAG CHEMIE, HOBOKEN, USA, vol. 57, no. 30, 25 June 2018 (2018-06-25), pages 9310 - 9314, XP072104373, ISSN: 1433-7851, DOI: 10.1002/ANIE.201803108
- HONGYU ZHONG; MAX R. FRIEDFELD; PAUL J. CHIRIK: "Syntheses and Catalytic Hydrogenation Performance of Cationic Bis(phosphine) Cobalt(I) Diene and Arene Compounds", ANGEWANDTE CHEMIE INTERNATIONAL EDITION, vol. 58, no. 27, 27 May 2019 (2019-05-27), Hoboken, USA, pages 9194 - 9198, XP072103204, ISSN: 1433-7851, DOI: 10.1002/anie.201903766
- HERNÁNDEZ-VALDÉS DANIEL, AVIGNON FRÉDÉRIC, MÜLLER PETER, MEOLA GIUSEPPE, PROBST BENJAMIN, FOX THOMAS, SPINGLER BERNHARD, ALBERTO R: "[Re(η 6 -arene) 2 ] + as a highly stable ferrocene-like scaffold for ligands and complexes", DALTON TRANSACTIONS, vol. 49, no. 16, 28 April 2020 (2020-04-28), Cambridge , pages 5250 - 5256, XP093081678, ISSN: 1477-9226, DOI: 10.1039/D0DT00731E

## Description

### RELATED APPLICATION DATA

The present application claims priority pursuant to Article 8 of the Patent Cooperation Treaty to United States Provisional Patent Application Serial Number 63/290,157 filed December 16, 2021.

### FIELD

The present invention relates to transition metal catalysts for asymmetric hydrogenation and, in particular, to methods of making cationic Co(I) complexes.

### BACKGROUND

The asymmetric hydrogenation of olefins is one of the most powerful reactions for the synthesis of single enantiomer products. Traditionally, Schrock-Osborn type catalysts have been employed for asymmetric hydrogenation. While effective, these catalysts carry a high cost, given the rhodium metal center. In view of these economic disadvantages, catalysts employing more abundant transition metals remain elusive.

### SUMMARY

The invention claimed provides methods for the facile synthesis of cationic cobalt complexes for asymmetric hydrogenation of alkenes. ANGEWANDTE CHEMIE, VERLAG CHEMIE, HOBOKEN, USA, vol. 58, no. 27, 27 May 2019 (2019-05-27), pages 9194-9198 discloses the preparation of cationic cobalt (I) complexes starting with the dimeric complex. Synthetic methods described herein, in some embodiments, provide cationic Co(I) precatalysts on a time scale and yield permitting catalytic evaluation of a significant number of enantiopure ligands. The method of making a cationic cobalt(I) complex comprises providing a precursor complex of the formula: 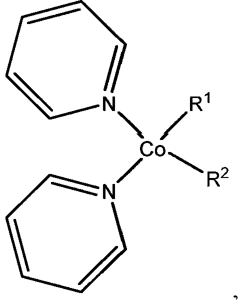 and
substituting ligands of the precursor complex with optically active bis(phosphine) ligand and arene ligand to provide the cationic cobalt(I) complex of formula: 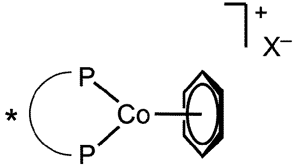 wherein R¹ and R² are independently selected from the group consisting of alkyl and heteroalkyl, and X⁻ is a counterion. As used herein, a heteroalkyl moiety includes one or more heteroatoms in the alkyl chain. In some embodiments, for example, heteroalkyl includes a silicon atom in the alkyl chain. Additionally, in some embodiments, one or both of the pyridine ligands of the precursor complex may be optionally substituted with one or more substituents. Substituents of one or both of the pyridine ligands can comprise any species not inconsistent with the technical objectives described herein. In some embodiments, substituents of the pyridine ligands can enhance crystallinity of the precursor complex, thereby facilitating handling of the precursor complex in synthetic methods described herein. In some embodiments, one or both of the pyridine ligands are independently substituted with alkyl substituents. One or more alkyl substituents may be the same or different between the two pyridine ligands. In some embodiments, the precursor complex is of the formula 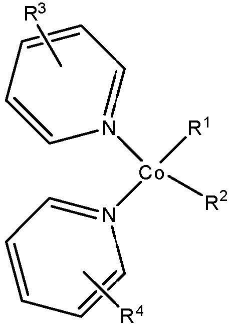 wherein R³ and R⁴ are independently alkyl. R³ and R⁴ can be the same or different.

Additionally, in some embodiments, various silver salts can be employed in oxidation of bis(phosphine)cobalt dialkyl complexes in the synthesis of cationic cobalt complexes described herein. In some embodiments, for example, silver salts of AgOTf, AgSbF₆ and/or AgBF₄ can replace ferrocenium salts in the oxidation.

Moreover, in some embodiments, the optically active bis(phosphine) ligand can displace the pyridine ligands of the precursor complex to provide an intermediate complex of the formula: 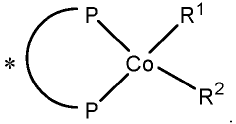 Additionally, R¹ and R² can be any alkyl or heteroalkyl consistent with displacement by arene ligand in the formation of the cationic Co(I) complex. In some embodiments, for example, at least one of R¹ and R² is -CH₂SiMe₃.

Disclosed, but not encompassed by the wording of the claims, a method of making a cationic cobalt (I) complex comprises providing a cationic cobalt(I) arene sandwich precursor complex, and substituting an arene ligand of the precursor complex with an optically active bis(phosphine) ligand to provide the cationic cobalt(I) complex of formula: 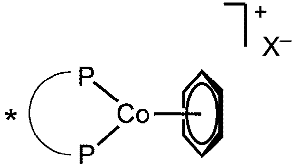 wherein X⁻ is a counterion. In some embodiments, the cationic cobalt(I) arene sandwich precursor complex is of the formula:

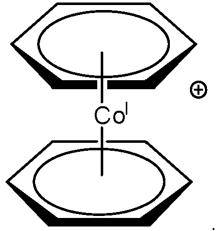

Disclosed, but not encompassed by the wording of the claims, methods of asymmetric alkene hydrogenation are described herein. In some embodiments, a method of asymmetric alkene hydrogenation comprises providing an alkene substrate, and hydrogenating the alkene in the presence of a cationic cobalt(I) catalyst to yield a single enantiomer reaction product, wherein the cationic cobalt(I) catalyst is derived from a precatalyst of the formula: 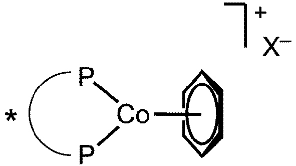 wherein 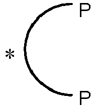 is optically active bis(phosphine) ligand, and X⁻ is a couterion. The precatalyst is synthesized by providing a precursor complex of the formula: 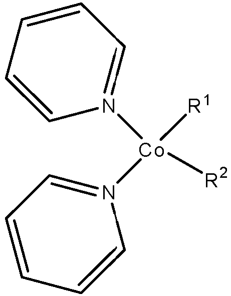 wherein one or both of the pyridine ligands are optionally subsituted with one or more alkyl substituents, and substituting ligands of the precursor complex with the optically active bis(phosphine) ligand and arene ligand, wherein R¹ and R² are independently selected from the group consisting of alkyl and heteroalkyl. In some embodiments, the alkene substrate is a pharmaceutical compound or a pharmaceutical precursor.

Disclosed, but not encompassed by the wording of the claims, a method of asymmetric alkene hydrogenation comprises providing an alkene substrate, and hydrogenating the alkene in the presence of a cationic cobalt(I) catalyst to yield a single enantiomer reaction product, wherein the cationic cobalt(I) catalyst is derived from a precatalyst of the formula: 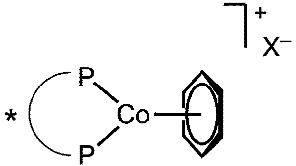 wherein 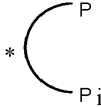 is optically active bis(phosphine) ligand, and X⁻ is a couterion. The precatalyst is synthesized by providing a cationic cobalt(I) arene sandwich precursor complex, and substituting an arene ligand of the precursor complex with the optically active bis(phosphine) ligand.

In methods described herein, the optically active bis(phosphine) ligand can be enantiopure. In some embodiments, the optically active bis(phosphine) ligand is selected from the group consisting of DuPhos, BenzP*, TangPhos, and DuanPhos. Moreover, X⁻ can be any counterion consistent with the technical objectives of the present application.

Synthetic methods described herein can yield cationic Co(I) complexes in an amount of at least 90% or 95%, in some embodiments. Additionally, reaction time for producing the cationic Co(I) complexes according to synthetic methods described herein can be less than 10 minutes. In some embodiments, reaction time for Co(I) catalyst production ranges from 1-10 minutes. Such high yields and fast reaction times permit catalytic evaluation of a significant number of enantiopure ligands when incorporated into the Co(I) architecture.

These and other embodiments are further described in the following detailed description.

### BRIEF DESCRIPTION OF THE DRAWINGS

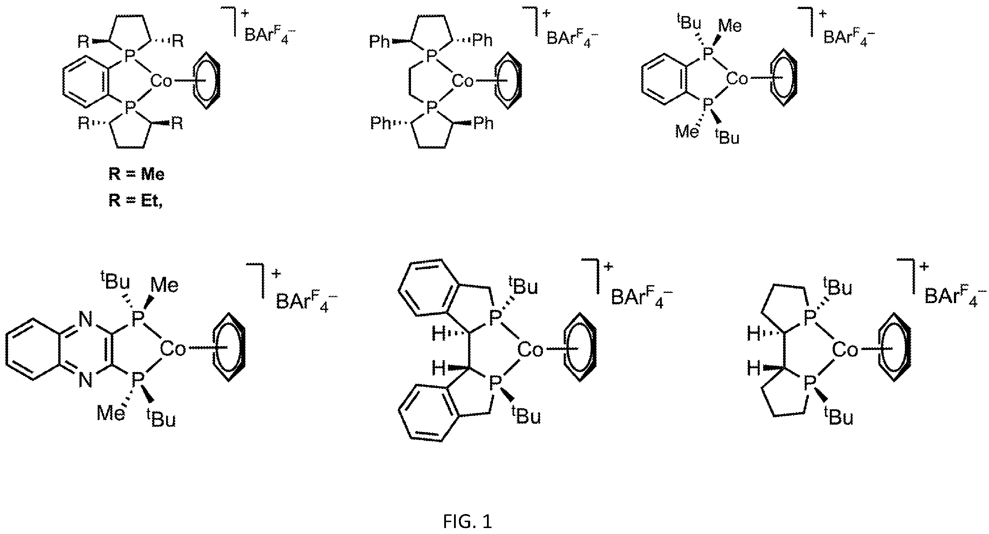

FIG. 1 illustrates various cationic Co(I) complexes synthesized according to methods described herein.

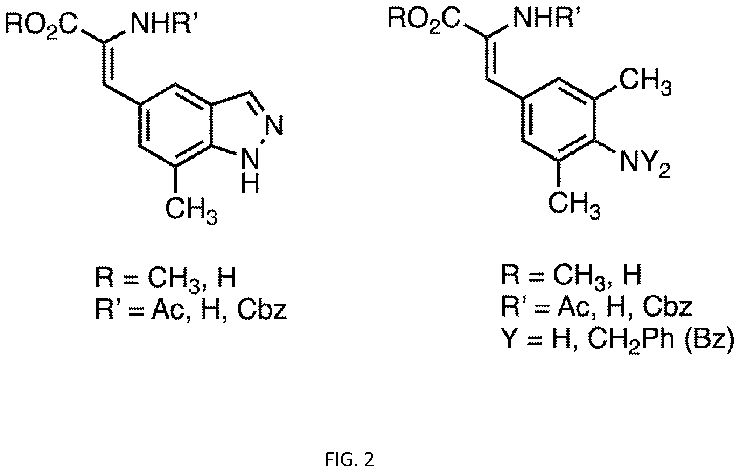

FIG. 2 illustrates various alkene substrates for asymmetric hydrogenation with Co(I) complexes described herein.
FIG. 3 illustrates a synthetic route for a Co(I) complex according to one embodiment of the invention.
FIG. 4 illustrates a synthetic route for a Co(I) complex according to one embodiment of the invention.
FIG. 5 illustrates a synthetic route for a Co(I) complex according to one embodiment of the invention.
FIG. 6 illustrates a synthetic route for a Co(I) complex according to one embodiment of the invention.
FIG. 7 illustrates a synthetic route for a Co(I) complex not encompassed by the wording of the claims.

### DETAILED DESCRIPTION

Embodiments described herein can be understood more readily by reference to the following detailed description and examples and their previous and following descriptions. Elements, apparatus and methods described herein, however, are not limited to the specific embodiments presented in the detailed description and examples. It should be recognized that these embodiments are merely illustrative of the principles of the present invention. Numerous modifications and adaptations will be readily apparent to those of skill in the art without departing from the scope of the invention.

### EXAMPLE 1 - Synthesis of Cationic Co(I) Complex for Asymmetric Hydrogenation

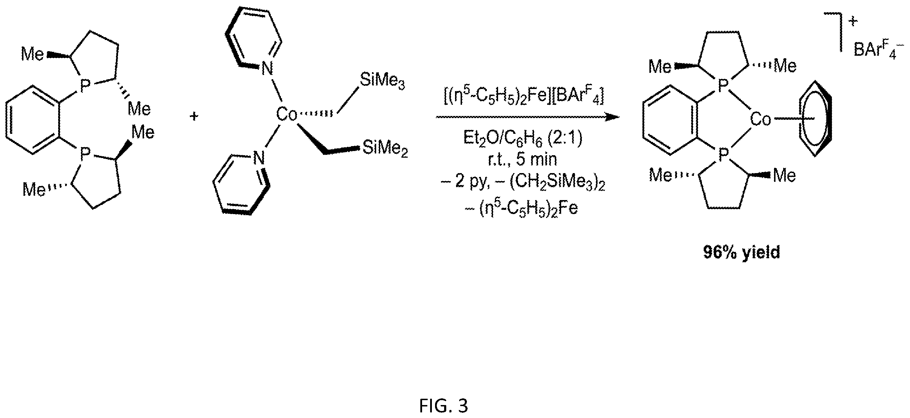

A Co(I) complex for asymmetric hydrogenation was prepared according to the reaction scheme of FIG. 3 and as follows.
**Preparation of [(*S*,*S*)-(^{Me}DuPhos)Co(η⁶-C₆H₆)][BAr^{F}₄].** In a nitrogen-filled glovebox, a 20 mL scintillation vial was charged with (py)₂Co(CH₂SiMe₃)₂ (0.097 g, 0.24 mmol) as a dark green semi-solid and 2 mL of diethyl ether were added. In a separate vial, (*S*,*S*)-^{Me}DuPhos (0.078 g, 0.25 mmol) was weighed out as a crystalline white solid and dissolved in diethyl ether. To the ethereal solution of (*S*,*S*)-^{Me}DuPhos was added (py)₂Co(CH₂SiMe₃)₂ dropwise. The dark green ethereal solution of (py)₂Co(CH₂SiMe₃)₂ instantly turned dark red when combined with bis(phosphine) and was stirred for 30 seconds at ambient temperature. Single-crystals of (*S*,*S*)-(^{Me}DuPhos)Co(CH₂SiMe₃)₂ deposited on the sides of the scintillation vial could be collected and used in the oxidatively-induced reductive elimination, however, routine precatalyst synthesis was done in one-pot. Finally, [(η⁵-C₅H₅)₂Fe][BAr^{F}₄] (0.251 g, 0.24 mmol) was weighed out as a deep blue solid and dissolved in 3 mL of a mixture of diethyl ether and benzene (2:1). When [(η⁵-C₅H₅)₂Fe][BAr^{F}₄] was added dropwise to the dark red ethereal solution of (*S*,*S*)-(^{Me}DuPhos)Co(CH₂SiMe₃)₂ (formed *in situ),* the deep blue color instantly gave way to bright yellow. The reaction mixture was stirred for 5 minutes at ambient temperature, at which point the solution became cloudy. Volatiles were removed under reduced pressure and the residue was washed with pentane to remove ferrocene and silane byproducts. The residue was taken up in diethyl ether and passed through a pad of Celite where it was then dried under reduced pressure to yield 0.301 g (96% yield) of **[(*S*,*S*)-(^{Me}DuPhos)Co(η⁶-C₆H₆)][BAr^{F}₄]** as an orange crystalline powder.

Anal Calcd for C₅₂H₄₆BCoF₂₄P₂: C, 51.48; H, 3.55. Found: C, 51.5; H, 3.2. ¹H NMR (500 MHz, THF-*d*₈, 23 °C): δ 7.79 (br m, 8H, *o*-B[(3,5-(CF₃)₂)C₆*H*₃]₄), 7.68 (br m, 2H, ^{iPr}DuPhos Ar-*H),* 7.59 (br m, 2H, ^{iPr}DuPhos Ar-*H*), 7.57 (br m, 4H, *p*-B[(3,5-(CF₃)₂)C₆*H*₃]₄), 6.38 (s, 6H, η⁶-C₆*H*₆), 2.65 (br m, 2H, ^{iPr}DuPhos C*H*), 2.34 (br m, 2H, ^{iPr}DuPhos C*H*), 2.31 (br m, 2H, ^{iPr}DuPhos C*H*₂), 2.20 (br m, 2H, ^{iPr}DuPhos C*H*₂), 1.80 (br m, 2H, ^{iPr}DuPhos C*H*₂), 1.55 (br m, 2H, ^{iPr}DuPhos C*H*₂), 1.37 (br m, 6H, ^{iPr}DuPhos C*H*₃), 0.86 (br m, 6H, ^{iPr}DuPhos C*H*₃). ¹³C{¹H} NMR (125.7 MHz, THF-*d*₈, 23 °C): δ 162.9 (q, ¹*J*_{B-C}= 50.1 Hz, B[(3,5-(CF₃)₂)*C*₆H₃]₄), 143.8 (app t, ^{iPr}DuPhos Ar), 135.8 (br s, B[(3,5-(CF₃)₂)*C*₆H₃]₄), 132.5 (s, ^{iPr}DuPhos Ar), 132.0 (app t, ^{iPr}DuPhos Ar), 130.2 (m, B[(3,5-(CF₃)₂)*C*₆H₃]₄), 125.7 (q, *¹J*_{C-F} *=* 272.5 Hz, B[(3,5-(CF₃)₂)C₆H₃]₄), 118.4 (br s, B[(3,5-(CF₃)₂)*C*₆H₃]₄), 93.6 (s, η⁶-C₆H₆), 45.9 (app t, ^{iPr}DuPhos CH), 42.6 (app t, ^{iPr}DuPhos CH), 37.2 (^{iPr}DuPhos *C*H₂), 36.8 (^{iPr}DuPhos *C*H₂), 17.9 (^{iPr}DuPhos *C*H₃), 13.9 (^{iPr}DuPhos *C*H₃). ³¹P{¹H} NMR (202.4 MHz, THF-*d*₈, 23 °C): δ 96.2 (s, 2P). ¹⁹F NMR (376.5 MHz, THF-*d*₈, 23 °C): δ -63.4 (s, B[(3,5-(C*F*₃)₂)C₆H₃]₄).

### EXAMPLE 2 - Synthesis of Cationic Co(I) Complex for Asymmetric Hydrogenation

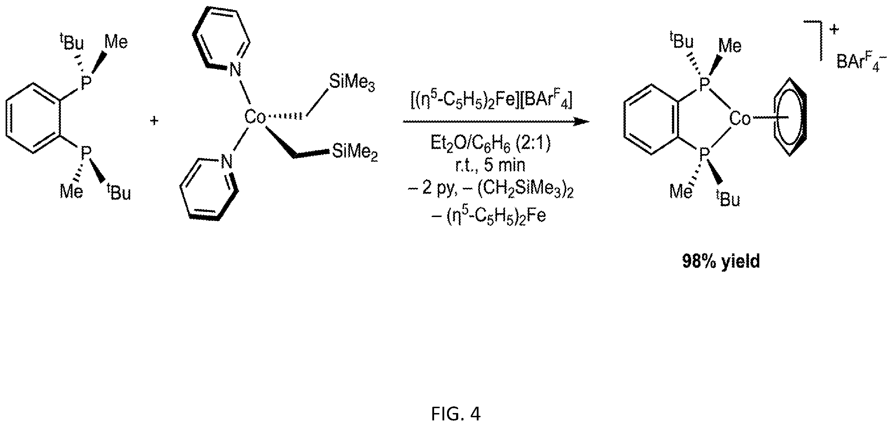

A Co(I) complex for asymmetric hydrogenation was prepared according to the reaction scheme of FIG. 4 and as follows.

**Preparation of [(*R*,*R*)-(BenzP*)Co(η⁶-C₆H₆)][BAr^{F}₄].** In a nitrogen-filled glovebox, a 20 mL scintillation vial was charged with (py)₂Co(CH₂SiMe₃)₂ (0.051 g, 0.13 mmol) as a dark green semi-solid and 2 mL of diethyl ether were added. In a separate vial, (*R*,*R*)-BenzP* (0.037 g, 0.13 mmol) was weighed out as a crystalline white solid and dissolved in diethyl ether. To the diethyl ether solution of (*R*,*R*)-BenzP* was added a diethyl ether solution of (py)₂Co(CH₂SiMe₃)₂ dropwise. A color change to bright orange was observed immediately and the mixture was stirred for an additional 30 seconds at ambient temperature. Finally, [(η⁵-C₅H₅)₂Fe][BAr^{F}₄] (0.136 g, 0.13 mmol) was weighed out as a deep blue solid and dissolved in a mixture of diethyl ether and benzene (2:1). When [(η⁵-C₅H₅)₂Fe][BAr^{F}₄] was added dropwise to the stirring ethereal solution of (*R*,*R*)-(BenzP*)Co(CH₂SiMe₃)₂ (formed *in situ),* the deep blue color instantly gave way to bright yellow. The reaction mixture was stirred for 5 minutes at ambient temperature, at which point the solution became cloudy. Volatiles were removed under reduced pressure and the residue was washed with pentane to remove ferrocene and silane byproducts. The residue was extracted into diethyl ether and passed through a pad of Celite where it was then dried under reduced pressure to yield 0.163 g (99% yield) of **[(*R,R*)-(BenzP*)Co(η⁶-C₆H₆)][BAr^{F}₄]** as an orange crystalline powder. Anal Calcd for C₅₄H₄₆BCoF₂₄P₂: C, 50.57; H, 3.62. Found: C, 46.07; H, 2.75. ¹H NMR (500 MHz, THF-*d*₈, 23 °C): δ ¹H NMR (500 MHz, THF-*d*₈, 23 °C): δ 7.79 (br m, 8H, *o*-B[(3,5-(CF₃)₂)C₆*H*₃]₄), 7.76 (br m, 2H, BenzP* Ar), 7.64 (br m, 2H, BenzP* Ar), 7.57 (br m, *p*-B[(3,5-(CF₃)₂)C₆*H*₃]₄), 6.38 (s, 6H, η⁶-C₆*H*₆), 1.89 (m, 6H, BenzP* C*H*₃), 0.93 (m, 18H, BenzP* C*H*₃). ¹³C{¹H} NMR (125.7 MHz, THF-*d*₈, 23 °C): δ 162.9 (q, ¹*J*_{B-C} = 51.4 Hz, B[(3,5-(CF₃)₂)*C*₆H₃]₄), 135.7 (br s, B[(3,5-(CF₃)₂)*C*₆H₃]₄), 131.9 (s, BenzP* Ar), 131.4 (app t, BenzP* Ar), 127.9 (q, ¹*J*_{C-F} = 269.9 Hz, B[(3,5-(*C*F₃)₂)C₆H₃]₄), 118.3 (br m, B[(3,5-(CF₃)₂)*C*₆H₃]₄), 93.3 (s, η⁶-*C*₆H₆), 36.8 (m, BenzP* *C*H₃), 27.4 (s, BenzP* C(*C*H₃)₃), 11.7 (s, BenzP* *C*(CH₃)₂). ³¹P{¹H} NMR (202.4 MHz, THF-*d*₈, 23 °C): δ 77.3 (s, 2P). ¹⁹F NMR (376.5 MHz, THF-*d*₈, 23 °C): δ -63.4 (s, B[(3,5-(C*F*₃)₂)C₆H₃]₄).

### EXAMPLE 3 - Synthesis of Cationic Co(I) Complex for Asymmetric Hydrogenation

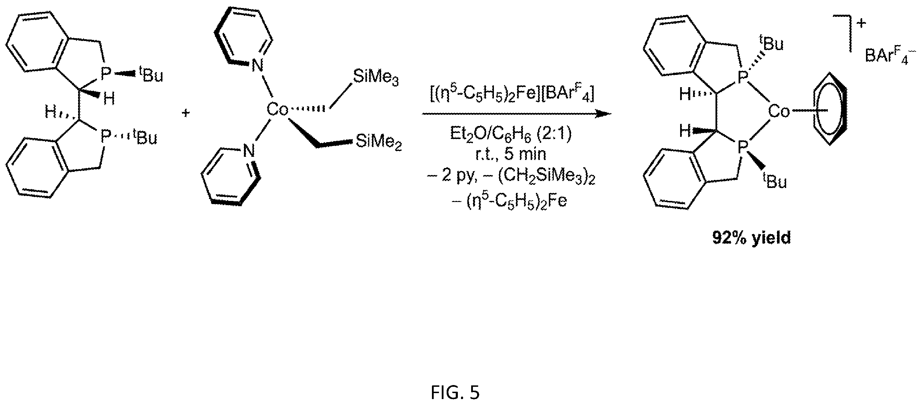

A Co(I) complex for asymmetric hydrogenation was prepared according to the reaction scheme of FIG. 5 and as follows.

**Preparation of [(*1S*,*1'S*,*1R*,*1'R*)-(DuanPhos)Co(η⁶-C₆H₆)][BAr^{F}₄]**. In a nitrogen-filled glovebox, a 20 mL scintillation vial was charged with (py)₂Co(CH₂SiMe₃)₂ (0.070 g, 0.18 mmol) as a dark green semi-solid and 2 mL of diethyl ether were added. In a separate vial, (*1S, 1 'S, 1R, 1* '*R*)-DuanPhos (0.075 g, 0.18 mmol) was weighed out as a crystalline orange solid and dissolved in diethyl ether. To the ethereal solution of (*1S, 1 'S, 1R, 1* '*R*)-DuanPhos was added a diethyl ether solution of (py)₂Co(CH₂SiMe₃)₂ dropwise. A color change to bright orange was observed and the reaction mixture was stirred an additional 30 seconds at ambient temperature. Finally, [(η⁵-C₅H₅)₂Fe][BAr^{F}₄] (0.188 g, 0.18 mmol) was weighed out as a deep blue solid and dissolved in a mixture of diethyl ether and benzene (2:1). The solution containing the [(η⁵-C₅H₅)₂Fe][BAr^{F}₄] was added dropwise to the stirring ethereal solution of (*1S, 1 'S, 1R, 1 'R*)-(DuanPhos)Co(CH₂SiMe₃)₂ (formed *in situ),* and a color change from deep blue to dark orange was observed. The reaction mixture was stirred for 5 minutes at ambient temperature, at which point the solution became cloudy. Volatiles were removed under reduced pressure and the residue was washed with pentane to remove ferrocene and silane byproducts. The residue was taken up in diethyl ether and passed through a pad of Celite where it was then dried under reduced pressure to yield 0.253 g (92% yield) of **[(*1S*,*1'S,1R,1'R*)-(DuanPhos)Co(η⁶-C₆H₆)][BAr^{F}₄]** as a dark orange crystalline powder. Anal Calcd for C₆₂H₅₀BCoF₂₄P₂: C, 50.41; H, 3.92. Found: C, 50.04; H, 3.51. ¹H NMR (500 MHz, THF-*d*₈, 23 °C): at 23 °C, spectra were broad and uninformative. ³¹P{¹H} NMR (202.4 MHz, THF-*d*₈, 23 °C): δ 127.2 (s, 2P). ¹⁹F NMR (376.5 MHz, THF-*d*₈, 23 °C): δ -63.5 (s, B[(3,5-(C*F*₃)₂)C₆H₃]₄).

### EXAMPLE 4 - Synthesis of Cationic Co(I) Complex for Asymmetric Hydrogenation

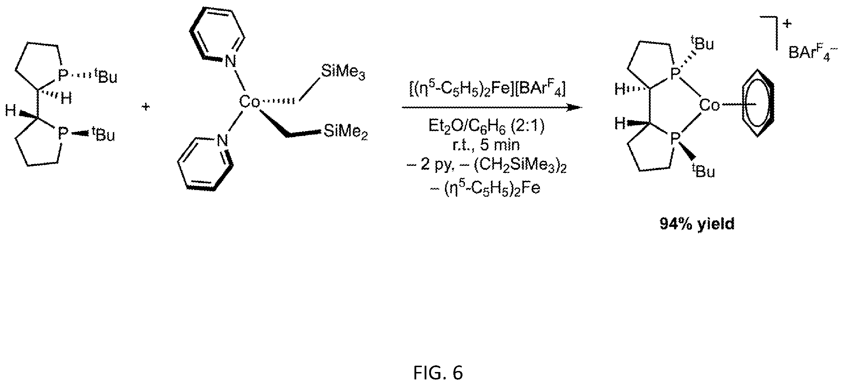

A Co(I) complex for asymmetric hydrogenation was prepared according to the reaction scheme of FIG. 6 and as follows.

**Preparation of [(*S,S',R,R'*)-(TangPhos)Co(η⁶-C₆H₆)][BAr^{F}₄].** In a nitrogen-filled glovebox, a 20 mL scintillation vial was charged with (py)₂Co(CH₂SiMe₃)₂ (0.070 g, 0.18 mmol) as a dark green semi-solid and 2 mL of diethyl ether were added. In a separate vial, (*S,S',R,R*')-TangPhos (0.075 g, 0.18 mmol) was weighed out as a crystalline orange solid and dissolved in diethyl ether. To the ethereal solution of (*S,S',R,R* ')-TangPhos was added (py)₂Co(CH₂SiMe₃)₂ dropwise. The dark green ethereal solution of (py)₂Co(CH₂SiMe₃)₂ instantly turned bright orange when combined with bis(phosphine) and was stirred for 30 seconds at ambient temperature. Finally, [(η⁵-C₅H₅)₂Fe][BAr^{F}₄] (0.188 g, 0.18 mmol) was weighed out as a deep blue solid and dissolved in a mixture of diethyl ether and benzene (2:1). When [(η⁵-C₅H₅)₂Fe][BAr^{F}₄] was added dropwise to the stirring ethereal solution of (*S,S',R,R'*)-(TangPhos)Co(CH₂SiMe₃)₂ (formed *in situ),* the deep blue color instantly gave way to dark orange. The reaction mixture was stirred for 5 minutes at ambient temperature, at which point the solution became cloudy. Volatiles were removed under reduced pressure and the residue was washed with pentane to remove ferrocene and silane byproducts. The residue was taken up in diethyl ether and passed through a pad of Celite where it was then dried under reduced pressure to yield 0.253 g (92% yield) of **[(*S,S',R,R'*)-(TangPhos)Co(η⁶-C₆H₆)][BAr^{F}₄]** as a dark orange crystalline powder. Anal Calcd for C₅₄H₅₀BCoF₂₄P₂: C, 50.41; H, 3.92. Found: C, 50.04; H, 3.51. ¹H NMR (500 MHz, THF-*d*₈, 23 °C): δ 7.78 (br m, 8H, *o*-B[(3,5-(CF₃)₂)C₆*H*₃]₄), 7.57 (br m, *p*-B[(3,5-(CF₃)₂)C₆*H*₃]₄), 6.26 (s, 6H, η⁶-C₆*H*₆), 2.43-2.31 (ov m, 4H, TangPhos CH + TangPhos C*H*₂), 2.09-1.94 (ov m, 6H, TangPhos C*H*₂), 1.63 (m, 2H, TangPhos C*H*₂), 1.17 (m, 18H, TangPhos C(C*H*₃)₃). ¹³C{¹H} NMR (125.7 MHz, THF-*d*₈, 23 °C): δ 162.9 (q, ¹*J*_{B-C}= 50.1 Hz, B[(3,5-(CF₃)₂)*C*₆H₃]₄), 135.7 (br s, B[(3,5-(CF₃)₂)*C*₆H₃]₄), 130.1 (br m, B[(3,5-(CF₃)₂)*C*₆H₃]₄), 125.7 (q, ¹*J*_{C-F}*=* 272.2 Hz, B[(3,5-(*C*F₃)₂)C₆H₃]₄), 118.3 (br m, B[(3,5-(CF₃)₂)*C*₆H₃]₄), 92.8 (s, η⁶-*C*₆H₆), 46.5 (app t, TangPhos CH), 37.4 (app t, TangPhos *C*H₂), 35.5 (app t, TangPhos *C*H₂), 33.6 (app t, TangPhos *C*H₂), 28.5 (s, TangPhos C(*C*H₃)₃), 28.2 (s, TangPhos *C*(CH₃)₃). ³¹P{¹H} NMR (202.4 MHz, THF-*d*₈, 23 °C): δ 121.7 (s, 2P). ¹⁹F NMR (376.5 MHz, THF-*d*₈, 23 °C): δ -63.4 (s, B[(3,5-(C*F*₃)₂)C₆H₃]₄).

### EXAMPLE 5 (not encompassed by the wording of the claims) - Synthesis of Cationic Co(I) Complex for Asymmetric Hydrogenation

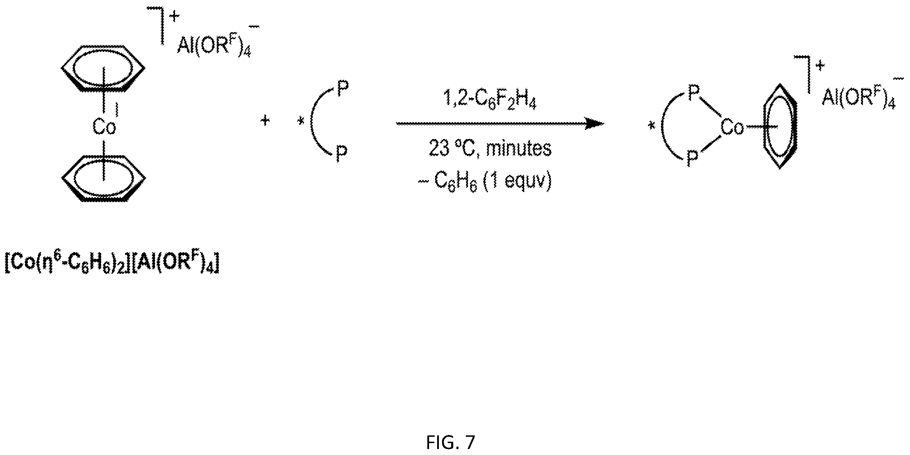

A Co(I) complex for asymmetric hydrogenation was prepared according to the reaction scheme of FIG. 7 and as follows.

This method utilizes a 20-electron arene 'sandwich' cation, (e.g. [Co(η⁶-C₆H₆)₂][X]) stabilized by either the weakly coordinating anion (WCA), [Al(OR^{F})₄]⁻ (R^{F} = C(CF₃)₃) developed by the group of Ingo Krossing, or commonly-used anions including BAr^{F}₄⁻, SbF₆⁻, PF₆⁻, and BF₄⁻. We have shown that combinations of [Co(η⁶-C₆H₆)₂][Al(OR^{F})₄] and optically-active bis(phosphines) including (*R*,*R*)-^{iPr}DuPhos, lead to air-stable, 18-electron bis(phosphine) cobalt cations similar to those described above with the key distinction being the composition of the anion. The reaction to generate the 18-electron cations must be carried out in 1,2-difluorobenzene given the poor stability of the 20-electron arene 'sandwich' complex [Co(η⁶-C₆H₆)₂][Al(OR^{F})₄] in coordinating solvents including THF. A representative example is given below:
In a nitrogen-filled glovebox, a 20 mL scintillation vial was charged with [Co(η⁶-C₆H₆)₂][Al(OR^{F})₄] (0.010 g, 8.5 µmol) and dissolved in 2 mL of 1,2-difluorobenzene to give an amber-colored solution. Separately, (*R,R*)-^{iPr}DuPhos (0.004 g, 8.6 µmol) was dissolved in 2 mL of benzene and added to [Co(η⁶-C₆H₆)₂][Al(OR^{F})₄] at ambient temperature resulting in an immediate color change from amber to bright yellow. Characterization of the product by multinuclear NMR spectroscopy in THF-*d*₈ gave nearly-identical shifts to the 18-electron cobalt complex [(*R,R*)-(^{iPr}DuPhos)Co(η⁶-C₆H₆)][BAr^{F}₄] with the exception of the ¹⁹F NMR spectrum which contained a single peak corresponding to the CF₃ groups of the aluminate anion. The activity of this complex was surveyed in the asymmetric hydrogenation of dehydro-sitagliptin where it maintained excellent enantioselectivity (96% ee) at 50 °C under 1000 psi of H₂.

### EXAMPLE 6 - Synthesis of Cationic Co(I) Complex for Asymmetric Hydrogenation

**Preparation of [(*R,R*)-(^{iPr}DuPhos)Co(η⁶-C₆H₆)][BAr^{F}₄].** In a nitrogen-filled glovebox, a 20 mL scintillation vial was charged with (3,5-(Me)₂-C₅H₃N)₂Co(CH₂SiMe₃)₂ (0.081 g, 0.18 mmol) as a dark green crystalline solid and dissolved in 2 mL of diethyl ether. In a separate vial, (*R,R*)-^{iPr}DuPhos (0.075 g, 0.18 mmol) was weighed out as a crystalline white solid and dissolved in diethyl ether. To the ethereal solution of (*R*,*R*)-^{iPr}DuPhos was added (3,5-(Me)₂-C₅H₃N)₂Co(CH₂SiMe₃)₂ dropwise. The dark green ethereal solution of (3,5-(Me)₂-C₅H₃N)₂Co(CH₂SiMe₃)₂ instantly turned bright orange when combined with bis(phosphine) and was stirred for 30 seconds at ambient temperature. Finally, [(η⁵-C₅H₅)₂Fe][BAr^{F}₄] (0.188 g, 0.18 mmol) was weighed out as a deep blue solid and dissolved in a mixture of diethyl ether and benzene (2:1). When [(η⁵-C₅H₅)₂Fe][BAr^{F}₄] was added dropwise to the stirring ethereal solution of (*R*,*R*)-(^{iPr}DuPhos)Co(CH₂SiMe₃)₂ (formed *in situ),* the deep blue color instantly gave way to bright yellow. The reaction mixture was stirred for 5 minutes at ambient temperature, at which point the solution became cloudy. Volatiles were removed under reduced pressure and the residue was washed with pentane to remove ferrocene and silane byproducts. The residue was taken up in diethyl ether and passed through a pad of Celite where it was then dried under reduced pressure to yield 0.251 g (98% yield) of **[(*R*,*R*)-(^{iPr}DuPhos)Co(η⁶-C₆H₆)][BAr^{F}₄]** as an orange-yellow crystalline powder. Characterization data were consistent with our previously-reported method involving chloride abstraction from [(*R*,*R*)-(^{iPr}DuPhos)Co(µ-Cl)]₂ with NaBAr^{F}₄.

Various embodiments of the invention have been described in fulfillment of the various objectives of the invention. It should be recognized that these embodiments are merely illustrative of the principles of the present invention. Numerous modifications and adaptations thereof will be readily apparent to those skilled in the art without departing from the scope of the invention as long as they fall within the scope of the appended claims.

## Claims

1. A method of making a cationic cobalt(I) complex comprising:
providing a precursor complex of the formula: 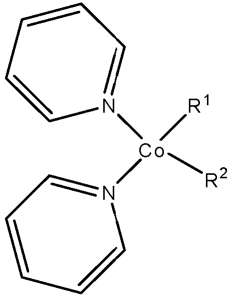 wherein one or both of the pyridine ligands are optionally subsituted with one or more substituents; and
substituting ligands of the precursor complex with optically active bis(phosphine) ligand and arene ligand to provide the cationic cobalt(I) complex of formula: 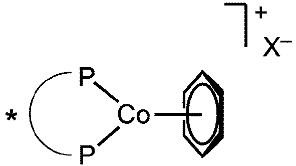 wherein R¹ and R² are independently selected from the group consisting of alkyl and heteroalkyl, and X⁻ is a counterion.

2. The method of claim 1, wherein the optically active bis(phosphine) ligand is enantiopure.

3. The method of claim 1, wherein the optically active bis(phosphine) ligand displaces the pyridine ligands of the precursor complex to provide an intermediate complex of the formula:

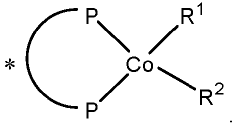

4. The method of claim 1, wherein the optically active bis(phosphine) ligand is selected from the group consisting of DuPhos, BenzP*, TangPhos, and DuanPhos.

5. The method of claim 1, wherein the precursor complex is of the formula 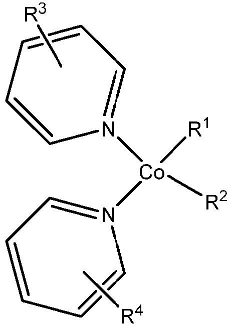 wherein R³ and R⁴ are independently alkyl.

## Patentansprüche

1. Eine Methode zur Bildung eines kationischen Kobalt(I)-Komplexes, bestehend aus:
was einen Vorläuferkomplex der Formel liefert: 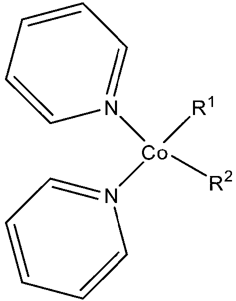
wobei einer oder beide der Pyridinliganden optional mit einem oder mehreren Substituenten ersetzt werden; und
indem Liganden des Vorläuferkomplexes durch optisch aktiven Bis(Phosphin)-Liganden und Aren-Liganden ersetzt werden, um den kationischen Kobalt(I)-Komplex der Formel zu erhalten: 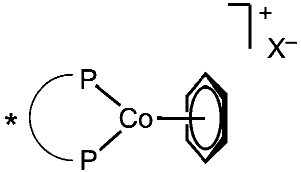
wobei R1 und R2 unabhängig voneinander aus der Gruppe aus Alkyl und Heteroalkyl ausgewählt werden, und X⁻ ein Counterion ist.

2. Die Methode des Anspruch 1, bei dem der optisch aktive Bis(Phosphin)-Ligand enantiopure ist.

3. Die Methode des Anspruchs 1, bei der der optisch aktive Bis(Phosphin)-Ligand die Pyridinliganden des Vorläuferkomplexes verdrängt, um einen Zwischenkomplex der Formel zu liefern:

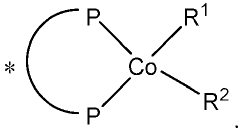

4. Die Methode von Anspruch 1, bei der der optisch aktive Bis(Phosphin)-Ligand aus der Gruppe aus DuPhos, BenzP*, TangPhos und DuanPhos ausgewählt wird.

5. Die Methode des Anspruch1, wobei der Vorläuferkomplex von der Formel ist 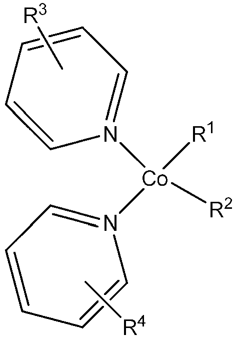 wobei R3 und R4 unabhängig voneinander Alkyl sind.

## Revendications

1. Une méthode pour créer un complexe cationique cobalt(I) comprenant :
fournissant un complexe précurseur de la formule : 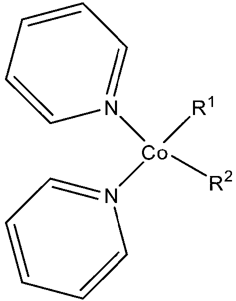
où un ou les deux ligands pyridine sont optionnellement remplacés par un ou plusieurs substituts ; et
en substituant les ligands du complexe précurseur par un ligand bis(phosphine) et un ligand d'arène optiquement actifs pour obtenir le complexe cationique cobalt(I) de formule : 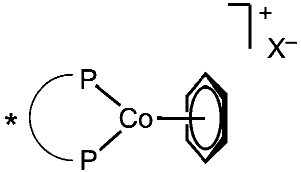
où R1 et R2 sont sélectionnés indépendamment du groupe constitué d'alkyle et d'hétéralkyle, et X⁻ est un contreion.

2. La méthode de la revendication 1, dans laquelle le ligand bis(phosphine) optiquement actif est énantiopure.

3. La méthode de la revendication 1, dans laquelle le ligand bis(phosphine) optiquement actif déplace les ligands pyridine du complexe précurseur pour fournir un complexe intermédiaire de la formule :

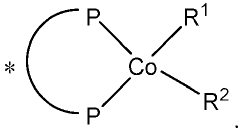

4. La méthode de la revendication 1, dans laquelle le ligand bis(phosphine) optiquement actif est sélectionné parmi le groupe composé de DuPhos, BenzP*, TangPhos et DuanPhos.

5. La méthode de la revendication 1, dans laquelle le complexe précurseur est de la formule 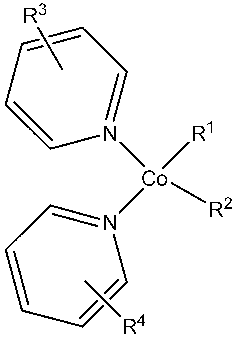 où R3 et R4 sont indépendamment alkylés.
